(19) Europäisches Patentamt
European Patent Office
Office européen des brevets

(11) Veröffentlichungsnummer: **0 030 688**
**B1**

# (12) EUROPÄISCHE PATENTSCHRIFT

(45) Veröffentlichungstag der Patentschrift: **28.09.83**

(51) Int. Cl.³: **C 07 D 409/12,** A 61 K 31/445

(21) Anmeldenummer: **80107648.0**

(22) Anmeldetag: **05.12.80**

(54) **Piperidinderivate von 3-Hydroxy-thiophen-2-carbonsäureestern, ihre Herstellung und diese enthaltende pharmazeutische Zubereitungen.**

(30) Priorität: **13.12.79 DE 2950064**

(43) Veröffentlichungstag der Anmeldung: **24.06.81 Patentblatt 81/25**

(45) Bekanntmachung des Hinweises auf die Patenterteilung: **28.09.83 Patentblatt 83/39**

(84) Benannte Vertragsstaaten: **AT BE CH DE FR GB IT LI NL SE**

(56) Entgegenhaltungen:
**DE-A-1 935 558**
**DE-A-2 242 629**
**DE-A-2 630 152**
**DE-A-2 720 613**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Frickel, Fritz-Frieder, Dr., Silvanerweg 7, D-6705 Deidesheim (DE)**
Erfinder: **Von Philipsborn, Gerda, Dr., Naechstenbacher Weg 35, D-6940 Weinheim (DE)**
Erfinder: **Mueller, Claus D., Dr., Odenwaldring 84, D-6806 Viernheim (DE)**
Erfinder: **Lenke, Dieter, Dr., Kekuleplatz 1, D-6700 Ludwigshafen (DE)**

**EP 0 030 688 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Piperidinderivate von 3-Hydroxy-thiophen-2-carbonsäureestern, ihre Herstellung und diese enthaltende pharmazeutische Zubereitungen

Die vorliegende Erfindung betrifft substituierte Piperidinoalkyläther von 3-Hydroxy-thiophen-2-carbonsäureestern und ihre physiologisch verträglichen Säureadditionssalze, Verfahren zu ihrer Herstellung und diese Verbindungen enthaltende pharmazeutische Zubereitungen, die bei der Behandlung von Herzrhythmusstörungen verwendet werden können.

In der DE-OS 2 630 152 werden beispielsweise Derivate des 1-Phenoxy-propanol-2 mit einem 4-(2-Pyridyl)-piperidin-4-ol-Rest mit antiarrhythmische Eigenschaften beschrieben. Weiterhin sind beispielsweise Phen- und Naphthoxypropanolamin-Derivate mit einem 4-Phenyl-piperidin-4-ol-rest mit kreislaufwirksamen, insbesondere blutdrucksenkenden, Eigenschaften aus der DE-OS 2 242 629 und Piperazin-Derivate von Thiophenestern mit blutdrucksenkenden Eigenschaften bekannt. Dem Fachmann ist bekannt, daß die bisher eingesetzten Mittel zur Bekämpfung von Herzrhythmusstörungen nicht immer befriedigen und diese häufig eine zu geringe therapeutische Breite aufweisen.

Es wurde gefunden, daß Verbindungen der allgemeinen Formel I

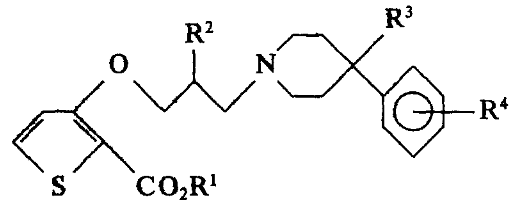

(I)

in der $R^1$ Methyl oder Ethyl, $R^2$ ein Wasserstoffatom oder eine Hydroxylgruppe, $R^3$ ein Wasserstoffatom, eine Hydroxyl- oder eine Carboethoxygruppe und $R^4$ ein Wasserstoffatom, ein Fluor-, Chlor- oder Bromatom, einen Trifluormethylrest oder eine Methoxygruppe bedeuten und ihre physiologisch verträglichen Säureadditionssalze wertvolle pharmakologische Eigenschaften aufweisen.

Dementsprechend sind als erfindungsgemäße Verbindungen der Formel I beispielsweise zu nennen:

3-[2-Hydroxy-3-(4-phenyl-4-hydroxy-piperidino)-propoxy]-2-thiophen-carbonsäuremethylester
3-[2-Hydroxy-3-(4-phenyl-4-hydroxy-piperidino)-propoxy]-2-thiophen-carbonsäureethylester
3-[3-(4-phenyl-4-hydroxy-piperidino)-propoxy]-2-thiophen-carbonsäuremethylester
3-[3-(4-phenyl-4-hydroxy-piperidino)-propoxy]-2-thiophencarbonsäureethylester
3-[2-Hydroxy-3-(4-(p-chlorphenyl)-4-hydroxy-piperidino)-propoxy]-2-thiophencarbonsäureethylester
3-[2-Hydroxy-3-(4-(p-chlorphenyl)-4-hydroxy-piperidino)-propoxy]-2-thiophencarbonsäuremethylester
3-[2-Hydroxy-3-(4-(p-fluorphenyl)-4-hydroxy-piperidino)-propoxy]-2-thiophencarbonsäuremethylester
3-[2-Hydroxy-3-(4-(p-fluorphenyl)-4-hydroxy-piperidino)-propoxy]-2-thiophencarbonsäureethylester
3-[2-Hydroxy-3-(4-(m-trifluormethylphenyl)-4-hydroxy-piperidino)-propoxy]-2-thiophencarbonsäuremethylester
3-[2-Hydroxy-3-(4-phenyl-4-carboethoxy-piperidino)-propoxy]-2-thiophencarbonsäuremethylester
3-[2-Hydroxy-3-(4-phenyl-piperidino)-propoxy]-2-thiophencarbonsäuremethylester
3-[2-Hydroxy-3-(4-phenyl-piperidino)-propoxy]-2-thiophencarbonsäureethylester

Die erfindungsgemäßen Verbindungen werden hergestellt, indem man ein Carboalkoxythiophen der Formel II

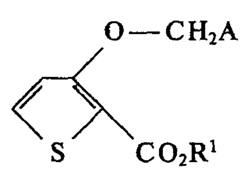

(II)

in der $R^1$ die für Formel I angegebene Bedeutung hat und A den Rest

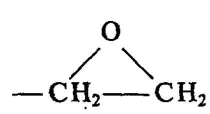 $-CH_2-CH_2-B$ oder 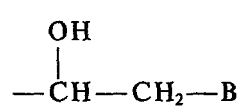

wobei B für eine nukleofuge Abgangsgruppe steht, bedeutet, mit einem Piperidinderivat der allgemeinen Formel III

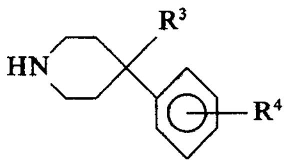

(III)

in der $R^3$ und $R^4$ die für Formel I angegebenen Bedeutungen haben, zweckmäßigerweise in einem Lösungsmittel und gegebenenfalls in Gegenwart eines säurebindenden Mittels in an sich bekannter Weise umsetzt und die erhaltene Verbindung gegebenenfalls in das Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurereste in Betracht, wie der p-Toluolsulfonsäure-, der p-Brombenzolsulfonsäure- oder der Methansulfonsäurerest.

Die Umsetzungen werden bei Temperaturen von 10 bis 120°C, d. h. bei Raumtemperaturen oder bei höheren Temperaturen, zweckmäßig bei Temperaturen von 50 bis 120°C durchgeführt. Die Umsetzungen können unter Atmosphärendruck oder in einem geschlossenen Gefäß unter erhöhtem Druck, gegebenenfalls unter Erwärmung auf den angegebenen Temperaturbereich, durchgeführt werden.

Die Ausgangsverbindungen können direkt, d. h. ohne Zugabe eines Verdünnungs- oder Lösungsmittels umgesetzt werden. Zweckmäßigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines aromatischen Kohlenwasserstoffes, wie Benzol oder eines Alkylbenzols, wie Toluol oder Xylol, oder eines gesättigten aliphatischen Kohlenwasserstoffes, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, oder von Dimethylsulfoxid oder in Gegenwart von Wasser oder in Mischungen der genannten Lösungsmittel, durchgeführt.

Bevorzugte Lösungsmittel bei der Umsetzung eines Epoxids der Formel (II), beispielsweise 1-(2-Carbomethoxy-thienyl-3-oxy)-2,3-epoxypropan oder 1-(2-Carboäthoxy-thienyl-3-oxy)-2,3-epoxypropan, mit einem Piperidinderivat der allgemeinen Formel (III) sind niedere Alkohole, insbesondere Isopropanol, wobei die Umsetzung bevorzugt bei Temperaturen von 50 bis 120°C und bei Normaldruck durchgeführt wird.

Bei der nukleophilen Substitution eines Restes B in einer Verbindung der Formel (II), beispielsweise von 1-(2-Carbomethoxy-thienyl-3-oxy)-3-brom-propan oder von 1-(2-Carbomethoxy-thienyl-3-oxy)-3-chlorpropan, sind als Lösungsmittel ein niederes aliphatisches Keton, wie Aceton, Diäthylketon, Methylisopropylketon oder Methylisobutylketon, ein cyclischer gesättigter Äther, insbesondere Tetrahydrofuran oder Dioxan, oder ein Dialkylformamid, wie Dimethylformamid und Temperaturen von 90 bis 180°C bevorzugt. Gegebenenfalls wird die Umsetzung in Gegenwart einer katalytischen Menge Natrium oder Kaliumjodid durchgeführt.

Es sei erwähnt, daß als Ausgangsverbindung der Formel II gegebenenfalls auch eine Mischung des Epoxids mit einem Halogenhydrin in Betracht kommt, da bei der technischen Herstellung der Ausgangsverbindungen der Formel II derartige Gemische unter Umständen entstehen können.

Bei einer zweckmäßigen Ausführungsform zur nukleophilen Substitution des Restes B durch das verwendete Piperidinderivat wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin, wie Pyridin oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuß verwendet. Gegebenenfalls ist es zweckmäßig, das zur Umsetzung verwendete Piperidin-Derivat (III) in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der Formel II können gemäß den in der DE-OS 1 935 558 beschriebenen Verfahren durch Alkylierung von 2-Carbomethoxy-3-hydroxy-thiophen oder 2-Carboethoxy-3-hydro-

xy-thiophen mit einem Epihalogenhydrin, einem $\alpha,\omega$-Dihalogen-2-propanol oder $\alpha,\omega$-Dihalogenpropan erhalten werden. Als Epihalogenhydrine kommen Epichlorhydrin, Epibromhydrin und Epijodhydrin, als $\alpha,\omega$-Dihalogen-2-propanole kommen insbesondere 1,3-Dichlor-2-propanol und 1,3-Dibrom-2-propanol und als $\alpha,\omega$-Dihalogenpropane insbesondere 1,3-Chlorbrompropan, 1,3-Dichlorpropan oder 1,3-Dibrompropan in Betracht.

Die Umsetzungen der 2-Carboalkoxy-3-hydroxy-thiophene zur Herstellung der Ausgangsverbindungen der Formel II werden zweckmäßigerweise bei Temperaturen von 50 bis 120°C und unter Normaldruck oder in einem geschlossenen Gefäß unter erhöhtem Druck durchgeführt. Die Umsetzungen werden zweckmäßigerweise in einem inerten Verdünnungs- oder Lösungsmittel, z. B. einem niederen aliphatischen Keton, wie Aceton, Methyläthylketon oder Methylisobutylketon, einem niederen Alkohol mit 1 bis 4 C-Atomen, wie Methanol, Äthanol, Propanol oder Butanol, einem niederen Alkylacetat, wie Methyl-, Äthyl- oder Propylacetat, einem Dialkylformamid, wie Dimethylformamid oder Diäthylformamid, oder Dimethylsulfoxid oder mit überschüssigem Alkylierungsmittel als Verdünnungs- oder Lösungsmittel durchgeführt. Bevorzugt werden die Umsetzungen in Gegenwart einer Base als säurebindendes Mittel vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin oder niedere Trialkylamine, wie Trimethyl- oder Triäthylamin. Dabei können die Basen im Verhältnis zum eingesetzten Alkylierungsmittel in katalytischer Menge oder in stöchiometrischer Menge bzw. in geringem Überschuß verwendet werden.

Bevorzugt werden die 2-Carboalkoxy-3-hydroxy-thiophene mit Epibromhydrin; 1,3-Dibrompropanol-2 oder 1,3-Dibrompropan in einem niederen aliphatischen Keton, insbesondere Aceton oder Methyl-isobutylketon, in Gegenwart von mindestens einem Moläquivalent Base, insbesondere Kaliumcarbonat, bezogen auf das Alkylierungsmittel, bei Temperaturen von 50 bis 80°C umgesetzt.

Weiterhin sei erwähnt, daß die Ausgangsverbindungen der Formel II mit einer Epoxygruppe oder mit Halogenhydrinstruktur durch einfache Säure-Base-Reaktion ineinander umgewandelt werden können. So läßt sich ein 1-(2-Carboalkoxy-thienyl-3-oxy)-2,3-epoxypropan mit der entsprechenden Halogenwasserstoffsäure in 1-(2-Carboalkoxy-thienyl-3-oxy)-3-halogen-isopropan-2-ol überführen, wobei als Verdünnungs- oder Lösungsmittel neben an sich üblichen Solventien vorzugsweise aliphatische oder cyclische Äther, wie Diäthyläther, Tetrahydrofuran oder Dioxan, oder ein niederer Alkohol, wie Methanol, Äthanol oder Propanol, verwendet werden. Andererseits können die 1-(2-Carboalkoxy-thienyl-3-oxy)-3-halogen-isopropan-2-ol-Verbindungen, insbesondere das 1-(2-Carbomethoxy-thienyl-3-oxy)-3-chlor-isopropan-2-ol und das 1-(2-Carbomethoxy-thienyl-3-oxy)-3-brom-isopropan-2-ol mit einer Base, wie einem Alkalimetallhydroxid, -carbonat, -hydrogencarbonat, -alkoholat oder -hydrid, einem organischen Amin, wie Pyridin, Piperidin oder einem tertiären aliphatischen Amin, wie Trimethylamin oder Triäthylamin, in 1-(2-Carboalkoxy-thienyl-3-oxy)-2,3-epoxypropan umgewandelt werden. Diese Reaktionen können bei Raumtemperatur durchgeführt werden oder durch Wärmezufuhr, z. B. durch Erwärmen auf 60 bis 120°C, beschleunigt oder abgeschlossen werden. Die Reaktion kann unter Atmosphärendruck oder in einem geschlossenen Gefäß unter erhöhtem Druck gegebenenfalls bei gleichzeitigem Erwärmen durchgeführt werden. Die Ausgangsstoffe für diese Umwandlung können zuvor isoliert oder im Reaktionsgemisch erzeugt und ohne weitere Isolierung und Reinigung unmittelbar weiterverarbeitet werden.

Die erhaltenen erfindungsgemäßen Verbindungen werden gegebenenfalls nach bekannter Verfahrensweise in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Apfelsäure, Zitronensäure, Salicylsäure, Adipinsäure oder Benzoesäure oder können aus »Fortschritte der Arzneimittelforschung«, Band 10, Seiten 224 bis 225, Birkhäuser-Verlag, Basel und Stuttgart, 1966, entnommen werden.

Die erfindungsgemäßen Verbindungen der Formel I, die eine Hydroxylgruppe in der aliphatischen Seitenkette am Kohlenstoffatom 2 tragen, weisen ein Chiralitätszentrum auf und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Bromcampher-8-sulfonsäure, in die optisch aktiven Antipoden getrennt werden können.

Die erfindungsgemäßen Verbindungen und ihre physiologisch verträglichen Säureadditionssalze sind stark antiarrhythmisch wirksam und eignen sich daher insbesondere zur Pharmakotherapie von Herzrhythmusstörungen.

Zur Bestimmung der antiarrhythmischen Wirksamkeit wird die Substanz Ratten (Stamm: Srague Dawley, Gewicht 200 bis 250 g) 45 min vor Beginn der Narkose oral appliziert. Die Tiere werden mit Thiobutabarbital-Natrium (100 mg/kg) intraperitoneal (i.p.) narkotisiert. Als arrhythmogene Substanz dient Aconitin, das 60 min nach Substanzapplikation intravenös (i.v.) infundiert wird (Dosierungsgeschwindigkeit: 0,005 mg/kg × min). Bei nicht behandelten Tieren (N = 52) treten nach 2,74 ± 0,07 min im EKG Arrythmien auf, deren Eintritt durch Antiarrhythmica dosisabhängig verzögert werden kann.

Aus der linearen Beziehung zwischen log Dosis (mg/kg) der Prüfsubstanz und der relativen

Verlängerung der Aconitininfusionsdauer (Δ%) wird die Dosis bestimmt, welche die Infusionsdauer um 50% verlängert (ED 50%).

Zur weiteren Charakterisierung der Substanz wird aus der in den Experimenten verwendeten dezimalgeometrischen Dosenfolge (Faktor $\sqrt[3]{10}$) die antiarrhythmische Wirkung der maximal tolerierten Dosis bestimmt. Ferner wird die Dosis ermittelt, bei welcher toxische Symptome (Veränderungen im Ausgangs-EKG, Cyanose, Krämpfe) auftreten. Darüber hinaus wird die antiarrhythmische Wirksamkeit der erfindungsgemäßen Verbindungen an isolierten linken Vorhöfen von männlichen Meerschweinchen (Stamm: Pirbright white, Gewicht 350 bis 450 g) bestimmt.

In einem Organbad (Volumen 125 ml) mit carbogengesättigter Tyrode (pH 7,4, Temperatur 32° C) suspendierte Vorhöfe sind mit 1,0 g vorbelastet und durch Rechteckimpulse von 1 Hz Grundrhythmus und doppelten Reizschwellenwerten (Rheobase: 0,2 bis 1,4 V, Chronaxie: 0,3 bis 0,5 msec) angetrieben.

Als Kriterium für antiarrhythmische Wirkung wird durch automatische kontinuierliche Frequenzerhöhung die Frequenz (Hz) bestimmt, bei welcher die Vorhöfe gerade noch folgen können (maximale Folgefrequenz). Aus der linearen Beziehung zwischen log Konzentration (mg/l) und der relativen Abnahme der maximalen Folgefrequenz (Δ%) wird die Konzentration berechnet, welche eine Abnahme der Folgefrequenz um 50% bewirkt (ED 50%).

Ferner wird aus der linearen Beziehung zwischen log Konzentration (mg/l) und der relativen Änderung der Kontraktionsamplitude (Δ%) als Maß für die negativ inotrope Wirkung die Konzentration ermittelt, welche eine Abnahme der Amplitude um 25% bewirkt (EC 25%).

Als Vergleichssubstanz dient das bekannte Antiarrhythmicum Procainamid.

Die Verbindung (Beispiel 1, vgl. Tabelle 1) ist an der Actonitinarrhythmie der Ratte 3 bis 4 mal stärker antiarrhythmisch wirksam als Procainamid. Ein weiterer Vorteil ist die im Vergleich mit Procainamid höhere Wirkung bei Applikation der höchsten tolerierten Dosis. Procainamid verlängert die Aconitininfusionsdauer um maximal 135%. Verbindung Beispiel 1 bewirkt eine maximale Zunahme um 296%.

Die als Quotient aus der toxischen und der antiarrhythmisch wirksamen Dosis (ED 50%) ermittelte therapeutische Breite dieser Verbindung ist 1,6mal größer als die von Procainamid.

Am isolierten Meerschweinchenvorhof (Tab. 2) wirkt diese neue Substanz 69mal so stark antiarrhythmisch wie Procainamid. Der Abstand zwischen antiarrhythmischer und negativ inotroper Wirkung — ersichtlich aus dem Quotienten EC 25% Kontraktionskraftabnahme : EC 50% maximale Folgefrequenzabnahme — ist wesentlich größer als bei Procainamid. Beispiel 1 verfügt damit auch am Meerschweinchenvorhof über die größere therapeutische Breite.

Tabelle 1

Antiarrhythmische Wirkung und Toxizität an der Ratte, Appl.: per os

| Substanz | Antiarrhythmische Wirkung an der Aconitinarrhythmie | | | | | Toxizität | |
|---|---|---|---|---|---|---|---|
| | Wirksame Dosis, mg/kg | | Maximale Wirkung[3] | | | | |
| | ED 50%[1] | R. W.[2] | Dosis | Δ %[4] | R. M. W.[5] | Dosis[6] | Q[7] |
| Beispiel 1 | 46,8 | 3,36 | 215 | 296 | 2,19 | 464 | 9,9 |
| Procainamid | 157 | 1,00 | 681 | 135 | 1,00 | 1000 | 6,4 |

[1]) Dosis, welche die Aconitininfusionsdauer um 50% verlängert.
[2]) R. W. = Relative Wirksamkeit: Procainamid = 1,00.
[3]) Wirkung der maximal tolerierten Dosis.
[4]) Verlängerung der Aconitininfusionsdauer Δ %.
[5]) R. M. W. = Relative maximale Wirkung: Procainamid = 1,00.
[6]) Dosis, nach deren Appl. die ersten toxischen Symptome beobachtet werden.
[7]) $Q = \frac{\text{toxische Dosis}}{\text{ED 50\%}}$.

Tabelle 2

Wirkung am isolierten Meerschweinchenvorhof

| Substanz | Maximale Folgefrequenz | | Kontraktionskraft | | Q[4]) |
|---|---|---|---|---|---|
| | EC 50%[1]) | R. W.[2]) | EC 25%[3]) | R. W. | |
| Beispiel 1 | 1,47 | 68,7 | 3,80 | 14,8 | 2,6 |
| Procainamid | 101 | 1,00 | 56,4 | 1,00 | 0,56 |

[1]) Konzentration (mg/l), welche eine 50%ige Abnahme der maximalen Folgefrequenz bewirkt.
[2]) R. W. = Relative Wirksamkeit: Procainamid = 1,0.
[3]) Konzentration (mg/l), welche eine Abnahme der Kontraktionskraft um 25% bewirkt.
[4]) $Q = \frac{\text{EC 25\% Kontraktionskraft}}{\text{EC 50\% Folgefrequenzabnahme}}$.

Gegenstand der vorliegenden Erfindung sind demnach auch therapeutische Mittel oder Zubereitungen, die neben üblichen Träger- und Verdünnungsmitteln eine Verbindung der Formel I oder deren physiologisch verträglichen Säureadditionssalze als Wirkstoff enthalten, sowie die Verwendung der neuen Verbindungen bei der Pharmakotherapie von Herzrhythmusstörungen.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln verarbeitet werden (vgl. L. G. Goodman, A. Gilman, The Pharmacological Basis of Therapeutics). Die so erhaltenen Präparate enthalten den Wirkstoff normalerweise in einer Menge von 0,001 und 99 Gew.-%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmittel wie Mais, Stärke oder Alginsäure, Bindemittel wie Stärke oder Gelatine, Gleitmittel wie Magnesiumstearat oder Talk und/oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Vanillin oder Orangenextrakt, enthalten. Sie können außerdem Suspendierstoffe, wie Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyäthylenglykol bzw. deren Derivaten herstellen.

Die Dosierung der erfindungsgemäßen Verbindungen hängt vom Alter, Zustand und Gewicht des Patienten sowie von der Applikationsart ab. In der Regel beträgt die tägliche Wirkstoffdosis 5 bis 100, vorzugsweise 10 bis 80 mg.

Die vorliegende Erfindung wird durch die nachfolgenden Ausführungsbeispiele näher erläutert.

## Herstellung von Ausgangsverbindungen

### Beispiel I

1-(2-Carbomethoxy-thienyl-3-oxy)-2,3-epoxypropan

800 g 2-Carbomethoxy-3-hydroxy-thiophen in 1,2 Liter Epichlorhydrin werden auf Rückflußtemperatur erhitzt, innerhalb einer Stunde mit 480 g einer 50%igen wäßrigen Natriumhydroxidlösung versetzt und das anfallende Azeotrop am Wasserabscheider getrennt. Nach beendetem Zutropfen beläßt man noch 15 min bei Rückflußtemperatur. Nach dem Abkühlen und Abdestillieren von ca. 1 Liter Epichlorhydrin wird das Reaktionsgemisch in Wasser gegossen und mit Methylenchlorid extrahiert. Die über Natriumsulfat getrockneten Extrakte erbringen nach der Destillation des Eindampfrückstandes 774 g 1-(2-Carbomethoxy-thienyl-3-oxy)-2,3-epoxypropan vom Sdp. 135 bis 142°C bei 0,2 bis 0,3 Torr, das langsam erstarrt:
Schmp.: 74 bis 75°C

$C_9H_{10}O_4S$ (214,2)

| | | | | |
|---|---|---|---|---|
| Ber.: | 50,5 C | 4,7 H | 29,9 O | 15,0 S |
| Gef.: | 50,7 C | 4,7 H | 29,5 O | 14,8 S |

### Beispiel II

1-(2-Carboäthoxy-thienyl-3-oxy)-2,3-epoxypropan

75 g 2-Carboäthoxy-3-hydroxy-thiophen, 50 ml Epibromhydrin und 100 g trockenes Kaliumcarbonat werden in 250 ml Aceton 10 Std. auf Rückflußtemperatur erhitzt. Nach dem Erkalten gießt man die gesamte Reaktionsmasse auf 3 Liter Eiswasser, extrahiert mit Methylenchlorid, wäscht die gesammelten Extrakte mit Wasser und trocknet über Natriumsulfat. Der nach dem Abdestillieren des Lösungsmittels verbleibende Eindampfrückstand wird fraktionierend destilliert. Man erhält 55 g 1-(2-Carboäthoxy-thienyl-3-oxy)-2,3-epoxypropan vom Sdp. 147 bis 153°C bei 0,2 Torr.

$C_{11}H_{12}O_4S$ (228,2)

| | | | |
|---|---|---|---|
| Ber.: | 57,9 C | 5,3 H | 14,0 S |
| Gef.: | 57,6 C | 5,6 H | 13,8 S |

### Beispiel III

1-(2-Carbomethoxy-thienyl-3-oxy)-3-chlor-propan-2-ol

4,0 g 1-(2-Carbomethoxy-thienyl-3-oxy)-2,3-epoxypropan werden in einem Gemisch aus 20 ml Methanol und 15 ml einer 4 N-ätherischen Salzsäure gelöst. Nach eintägigem Stehen werden die flüchtigen Bestandteile abdestilliert und der Eindampfrückstand an Kieselgel mit Methylenchlorid chromatographiert. Man erhält 2,1 g NMR-spektroskopisch reines 1-(2-Carbomethoxy-thienyl-3-oxy)-3-chlor-propan-2-ol.

$^1$H-NMR-Spektrum ($CDCl_3$, TMS intern):
=2,7 (d, J=5 Hz, 1H); 3,2 (d, J=5 Hz, 1 H);
5,3 (5,0 H); 5,8 (m, 3 H); 6,3 (5,3 H); 6,3 (m, 2 H)

### Beispiel IV

1-(2-Carbomethoxy-thienyl-3-oxy)-3-chlorpropan

79 g 2-Carboäthoxy-3-hydroxy-thiophen, 118 g 1,3-Bromchlorpropan und 104 g trockenes Kaliumcarbonat werden in 1 Liter Aceton 8 Stunden auf Rückflußtemperatur erhitzt. Nach dem Erkalten wird abfiltriert, der Filterrückstand mit Aceton nachgewaschen. Der verbleibende halbkristalline Rückstand wird aus Toluol umkristallisiert und erbringt 99 g 1-(2-Carbomethoxy-thienyl-3-oxy)-3-chlorpropan vom Schmp. 70 bis 71°C.

$C_9H_{11}ClO_3S$ (234,6)

| | | | | |
|---|---|---|---|---|
| Ber.: | 46,1 C | 4,7 H | 15,1 Cl | 13,7 S |
| Gef.: | 46,4 C | 4,5 H | 15,0 Cl | 13,9 S |

Herstellung von erfindungsgemäßen Verbindungen

Beispiel 1

100 g 1-(2-Carbomethoxy-thienyl-3-oxy)-2,3-epoxypropan und 82 g 4-Hydroxy-4-phenyl-piperidin werden in 400 ml Isopropanol 2 Stunden auf Rückflußtemperatur erhitzt. Der nach dem Abdestillieren verbleibende Rückstand wird aus Äthanol umkristallisiert und erbringt 145 g 3-[2-Hydroxy-3-(4-phenyl-4-hydroxy-piperidino)-propoxy]-2-thiophencarbonsäuremethylester vom Schmp. 153 – 154° C.

$C_{20}H_{25}NO_5S$ (391,2)

| | | | | | |
|---|---|---|---|---|---|
| Ber.: | 61,4 C | 6,4 H | 3,6 N | 20,4 O | 8,2 S |
| Gef.: | 61,4 C | 6,4 H | 3,5 N | 20,4 O | 8,2 S |

Beispiel 2

In einem Autoklaven werden 2,5 g 1-(2-Carbomethoxy-thienyl-3-oxy)-3-chlor-propan-2-ol und 1,9 g 4-Hydroxy-4-phenylpiperidin in 50 ml Dioxan 10 Std. auf 120° C erhitzt. Nach dem Abdestillieren der flüchtigen Anteile im Vakuum wird der hochviskose Rohaustrag in Äther-2 N Schwefelsäure verteilt, die Wasserphase vorsichtig mit 4 N-Natronlauge alkalisch gestellt und abschließend mit Äther extrahiert. Nach dem Trocknen der organischen Phase über Magnesiumsulfat wird vom Lösungsmittel befreit und der verbleibende Rückstand, wie in Beispiel 1 beschrieben, zweimal aus Äthanol umkristallisiert. Man erhält 2,0 g 3-[2-Hydroxy-3-(4-phenyl-4-hydroxy-piperidino)-propoxy]-2-thiophencarbonsäuremethylester vom Schmp. 150 – 152° C, das mit dem Material aus Beispiel 1 identisch ist.

Beispiel 3

4,7 g 1-(2-Carbomethoxy-thienyl-3-oxy)-3-chlorpropan, 3,5 g 4-Hydroxy-4-phenyl-piperidin und 2,7 g Natriumcarbonat werden in 30 ml N,N-Dimethylformamid 20 Std. auf Rückflußtemperatur erhitzt. Nach dem Erkalten wird zwischen Wasser und Methylenchlorid verteilt und der nach dem Eindampfen der organischen Phase im Vakuum verbleibende Rückstand in wenig Methanol gelöst und tropfenweise mit ätherischer Salzsäure versetzt. Das ausgefallene Kristallisat wird abgesaugt, mit Äther gewaschen und getrocknet. Man erhält 6,1 g 3-[3-(4-phenyl-4-hydroxy-piperidino)-propoxy]-2-thiophencarbonsäuremethylester-hydrochlorid-hydrat vom Schmp. 122 bis 123° C.

$C_{20}H_{28}ClO_5SN$ (430,9)

| | | | | | | |
|---|---|---|---|---|---|---|
| Ber.: | 55,7 C | 6,5 H | 18,6 O | 7,4 S | 3,3 N | 8,2 Cl |
| Gef.: | 55,8 C | 6,5 H | 18,7 O | 7,6 S | 3,3 N | |

Die in der nachfolgenden Tabelle angegebenen Verbindungen werden aus 1-(2-Carbomethoxy-thienyl-3-oxy)-2,3-epoxypropan bzw. 1-(2-Carboäthoxy-thienyl-3-oxy)-2,3-epoxypropan und den entsprechenden Piperidinen, wie in Beispiel 1 beschrieben, erhalten.

Tabelle 3

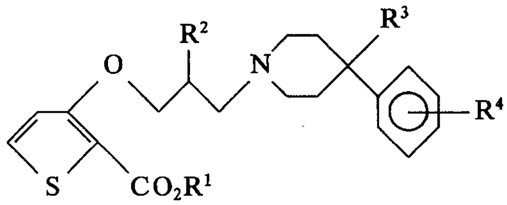

| Beispiel Nr. | $R^1$ | $R^2$ | $R^3$ | $R^4$ | Salzform | Schmp. °C |
|---|---|---|---|---|---|---|
| 4 | $C_2H_5$ | OH | OH | p-Cl | HCl | 174–175 |
| 5 | $CH_3$ | OH | OH | p-F | HCl | 154–155 |
| 6 | $CH_3$ | OH | OH | p-Cl | HCl | 192 (Zers.) |
| 7 | $CH_3$ | OH | OH | m-$CF_3$ | HCl | 154–156 |
| 8 | $C_2H_5$ | OH | OH | H | HCl | 136–138 |
| 9 | $CH_3$ | OH | $CO_2C_2H_5$ | H | HCl | 223–225 |
| 10 | $CH_3$ | OH | H | H | – | 115 |

Tabelle 3 (Fortsetzung)

| Beispiel Nr. | Analyse | | | | | | |
|---|---|---|---|---|---|---|---|
| 4 | Ber.: | 53,0 C | 5,7 H | 16,8 O | 2,9 N | 14,9 Cl | |
| | Gef.: | 52,9 C | 5,7 H | 16,9 O | 2,9 N | 15,1 Cl | |
| 5 | Ber.: | 53,9 C | 5,7 H | 3,1 N | 7,2 S | | |
| | Gef.: | 53,9 C | 5,6 H | 2,9 N | 7,2 S | | |
| 6 | Ber.: | 52,0 C | 5,5 H | 3,0 N | 6,9 S | 15,3 Cl | |
| | Gef.: | 52,4 C | 5,4 H | 3,1 N | 7,0 S | 15,5 Cl | |
| 7 | Ber.: | 51,0 C | 4,9 H | 2,8 N | 6,5 S | 11,5 F | 7,1 Cl |
| | Gef.: | 50,0 C | 5,0 H | 2,7 N | 6,6 S | 11,5 F | 7,0 Cl |
| 8 | Ber.: | 57,1 C | 6,4 H | 18,1 O | 3,2 N | 7,3 S | 8,0 Cl |
| | Gef.: | 57,0 C | 6,4 H | 18,0 O | 3,3 N | 7,2 S | 7,9 Cl |
| 9 | Ber.: | 57,1 C | 6,2 H | 19,8 O | 2,9 N | 6,6 S | 7,3 Cl |
| | Gef.: | 57,0 C | 6,1 H | 19,1 O | 3,1 N | 7,1 S | 6,9 Cl |
| 10 | Ber.: | 64,0 C | 6,7 H | 3,7 N | 8,5 S | | |
| | Gef.: | 64,0 C | 6,8 H | 3,8 N | 8,6 S | | |

Tabelle 3 (Fortsetzung)

| Beispiel Nr. | Name |
|---|---|
| 4 | 3-[2-Hydroxy-3-(4-(p-chlorphenyl)-4-hydroxy-piperidino)-propoxy]-2-thiophencarbonsäureäthylester |
| 5 | 3-[2-Hydroxy-3-(4-(p-fluorphenyl)-4-hydroxy-piperidino)-propoxy]-2-thiophencarbonsäuremethylester |
| 6 | 3-[2-Hydroxy-3-(4-(p-chlorphenyl)-4-hydroxy-piperidino)-propoxy]-2-thiophencarbonsäuremethylester |
| 7 | 3-[2-Hydroxy-3-(4-(m-trifluormethylphenyl)-4-hydroxy-piperidino)-propoxy]-2-thiophencarbonsäuremethylester |
| 8 | 3-[2-Hydroxy-3-(4-phenyl-4-hydroxy-piperidino)-propoxy]-2-thiophencarbonsäureäthylester |
| 9 | 3-[2-Hydroxy-3-(4-phenyl-4-carboäthoxy-piperidino)-propoxy]-2-thiophencarbonsäuremthylester |
| 10 | 3-[2-Hydroxy-3-(4-phenyl-piperidino)-propoxy]-2-thiophencarbonsäuremethylester |

III. Formulierungsbeispiele, die in üblicher Weise hergestellt werden

1. Tabletten

| | | |
|---|---|---|
| a) | Ein Wirkstoff der Formel I | 5 mg |
| | Lactose | 200 mg |
| | Methylcellulose | 15 mg |
| | Maisstärke | 50 mg |
| | Talkum | 11 mg |
| | Magnesiumstearat | 4 mg |
| b) | Ein Wirkstoff der Formel I | 20 mg |
| | Lactose | 178 mg |
| | Avicel® | 80 mg |
| | Polywachs 6000 | 20 mg |
| | Magnesiumstearat | 2 mg |
| c) | Ein Wirkstoff der Formel I | 50 mg |
| | Polyvinylpyrrolidon (mittl. M.G. 25 000) | 170 mg |
| | Polyäthylenglykol (mittl. M.G. 4000) | 14 mg |
| | Hydroxypropylmethylcellulose | 40 mg |
| | Talkum | 4 mg |
| | Magnesiumstearat | 2 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mitt. M.G. 4000), Hydroxypropylmethylcellulose, Talkum und Magnesiumstearat vermischt und zu Tabletten à 280 mg verpreßt.

**Patentansprüche**

1. Verbindungen der allgemeinen Formel I

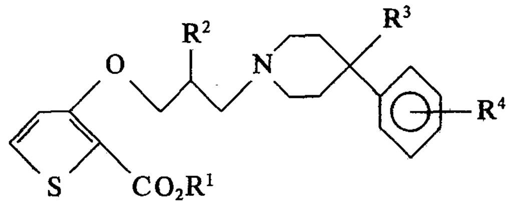

(I)

in der $R^1$ Methyl oder Ethyl, $R^2$ ein Wasserstoffatom oder eine Hydroxylgruppe, $R^3$ ein Wasserstoffatom, eine Hydroxyl- oder eine Carboethoxygruppe und $R^4$ ein Wasserstoffatom, ein Fluor-, Chlor- oder Bromatom, einen Trifluormethylrest oder eine Methoxygruppe bedeuten, und ihre physiologisch verträglichen Säureadditionssalze.

2. 3-[2-Hydroxy-3-(4-Phenyl-4-hydroxy-piperidino)-propoxy]-2-thiophencarbonsäuremethylester und dessen physiologisch verträglichen Säureadditionssalze.

3. Verfahren zur Herstellung von Verbindungen der Formel I nach Anspruch 1, dadurch gekennzeichnet, daß man ein Carboalkoxythiophen der Formel II

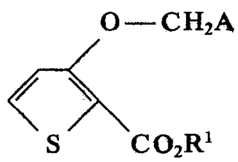

(II)

in der $R^1$ die für Formel I angegebenen Bedeutungen hat und A den Rest

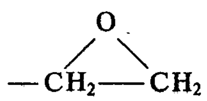 $-CH_2-CH_2-B$ oder 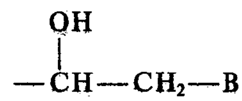

wobei B für eine nukleofuge Abgangsgruppe steht, bedeutet, mit einem Piperidinderivat der allgemeinen Formel III

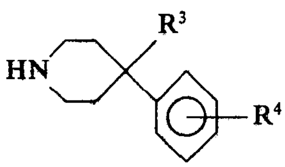

(III)

in der $R^3$ und $R^4$ die für Formel I angegebenen Bedeutungen haben, zweckmäßigerweise in einem Lösungsmittel und gegebenenfalls in Gegenwart eines säurebindenden Mittels in an sich bekannter Weise bei 10 bis 120°C umsetzt und die erhaltene Verbindung gegebenenfalls in das Säureadditionssalz einer physiologisch verträglichen Säure überführt.

4. Therapeutisches Mittel, gekennzeichnet durch einen Gehalt an einer Verbindung der Formel I oder ihres physiologisch verträglichen Säureadditionssalzes als Wirkstoff neben üblichen Trägerstoffen und Verdünnungsmitteln.

5. Verbindung der Formel I nach Anspruch 1, zur Verwendung bei der Pharmakotheraphie von Herzrhythmusstörungen.

**Claims**

1. A compound of the general formula I

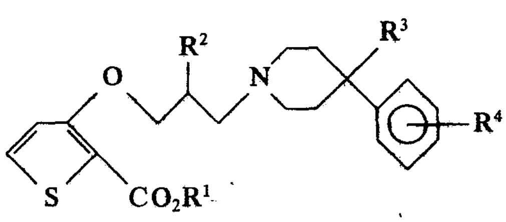

(I)

where $R^1$ is methyl or ethyl, $R^2$ is hydrogen or hydroxyl, $R^3$ is hydrogen, hydroxyl or carboethoxy, and $R^4$ is hydrogen, fluorine, chlorine or bromine, trifluoromethyl or methoxy, and its physiologically tolerated addition salts with acids.

2. 3-[2-Hydroxy-3-(4-phenyl-4-hydroxy-piperidino)-propoxy]-thiophene-2-carboxylic acid methyl ester and its physiologically tolerated addition salts with acids.

3. A process for the preparation of a compound of the formula I as claimed in claim 1, wherein a carboalkoxythiophene of the formula II

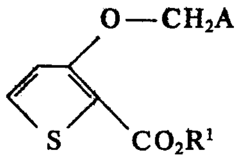

(II)

where $R^1$ has the meaning given for formula I and A is

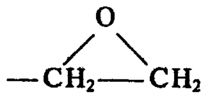 $-CH_2-CH_2-B$ or 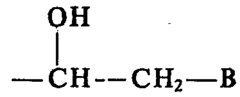

B being a nucleofugic leaving group, is reacted with a piperidine derivative of the general formula III

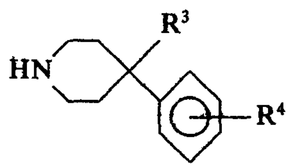

(III)

where $R^3$ and $R^4$ have the meanings given for formula I, in a conventional manner at 10 to 120°C, advantageously in a solvent and in the presence or absence of an acid acceptor and, if desired, the resulting compound is converted into an addition salt with a physiologically tolerated acid.

4. A therapeutic agent containing a compound of the formula I, or a physiologically tolerated acid addition salt thereof, as the active compound, in addition to conventional carriers and diluents.

5. A compound of the formula I as claimed in claim 1 for use in the pharmacotherapy of cardiac arrythmias.

**Revendications**

1. Composés de formule générale I

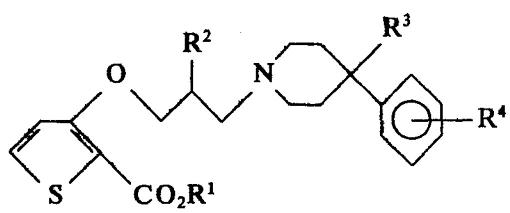

(I)

dans laquelle $R^1$ représente méthyle ou éthyle, $R^2$ un atome d'hydrogène ou un groupe hydroxyle, $R^3$ un atome d'hydrogène, un groupe hydroxyle ou un groupe carboéthoxy et $R^4$ un atome d'hydrogène, un atome de fluor, de chlore ou de brome, un reste trifluorométhyle ou un groupe méthoxy et leurs sels d'addition d'acide compatibles physiologiquement.

2. Ester méthylique d'acide 3-[2-hydroxy-3-(4-phényl-4-hydroxy-pipéridino)-propoxy]-2-thiophène-carboxylique et ses sels d'addition d'acide compatibles physiologiquement.

3. Procédé de préparation de composés de formule I selon la revendication 1, caractérisé par le fait qu'on fait réagir, de manière connue en soi, entre 10 et 120°C, de préférence dans un solvant et éventuellement en présence d'un agent liant les acides, un carboalcoxythiophène de formule II

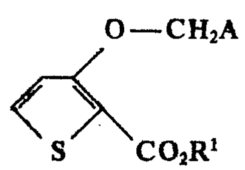

(II)

dans laquelle $R^1$ a la signification donnée pour la formule I et A représente le reste

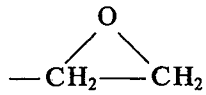    $-CH_2-CH_2-B$ ou 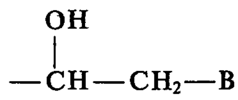

B représentant un groupe de départ nucléofuge, avec un dérivé de pipéridine de formule générale III

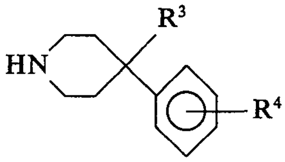

(III)

dans laquelle $R^3$ et $R^4$ ont les significations données pour la formule I, et l'on transforme le composé obtenu en le sel d'addition d'un acide compatible physiologiquement.

4. Agent thérapeutique, caractérisé par le fait qu'il contient, comme principe actif, un composé de formule I ou son sel d'addition d'acide compatible physiologiquement, outre les véhicules et diluants usuels.

5. Composé de formule I selon la revendication 1 pour l'utilisation en pharmacothéraphie des troubles du rythme cardiaque.